(19) Europäisches Patentamt
European Patent Office
Office européen des brevets

(11) **EP 3 783 360 A1**

(12) **EUROPÄISCHE PATENTANMELDUNG**

(43) Veröffentlichungstag:
**24.02.2021  Patentblatt 2021/08**

(51) Int Cl.:
***G01N 33/00*** *(2006.01)*

(21) Anmeldenummer: **20184942.9**

(22) Anmeldetag: **09.07.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **22.07.2019   DE 102019210818**

(71) Anmelder:
• **Liu, Jin-Chen**
  **85221 Dachau (DE)**

• **Goisser, Michael Martin**
  **85356 Freising (DE)**

(72) Erfinder:
• **Liu, Jin-Chen**
  **85221 Dachau (DE)**
• **Goisser, Michael Martin**
  **85356 Freising (DE)**

(74) Vertreter: **Grünecker Patent- und Rechtsanwälte PartG mbB**
**Leopoldstraße 4**
**80802 München (DE)**

(54) **VORRICHTUNG UND VERFAHREN ZUR MESSUNG DER TRANSPIRATION VON PFLANZEN**

(57)     Die Erfindung bezieht sich auf eine Vorrichtung (100) und ein Verfahren zum Ermitteln einer Transpirationsrate eines Objekts (1), insbesondere eines Pflanzenblatts oder einer Pflanzen-Nadel. Dabei wird eine erste Temperaturdifferenz zwischen den Temperaturen an der Oberfläche des Objekts (1) und an der Oberfläche eines Referenzkörpers (2) ermittelt, sowie eine zweite Temperaturdifferenz zwischen den Temperaturen an der Oberfläche des Objekts (1) bzw. an der Oberfläche des Referenzkörpers (2) und an einem davon beabstandeten Messpunkt (M1, M2), um daraus schließlich die Transpirationsrate des Objekts zu berechnen.

FIG. 1

## Beschreibung

Die Erfindung bezieht sich auf eine Vorrichtung zum Ermitteln einer Transpirationsrate eines Objekts, beispielsweise eines Pflanzenblatts oder einer Pflanzen-Nadel, sowie auf ein entsprechendes Verfahren.

Transpiration ist ein essenzieller physiologischer Vorgang in Pflanzen. Die Transpirationsrate (d. h. die Menge des über einen bestimmten Zeitraum hinweg transpirierten Wassers) ist eine Basisgröße im Wasserhaushalt der Pflanzen und daher von großem Interesse für Anwendung z.B. in der Bewässerungssteuerung, in Smart Farming und in der Forschung. Transpirationsraten sind stark abhängig von Pflanzenart und Bewässerungszustand der Pflanze. Typische Transpirationsraten sind im Bereich zwischen 0,2 - 4,0 mmol*m^-2*s^-1 für Buche und 0,1 - 1,0 mmol*m^-2*s^-1 im Fall der Fichte.

Transpiration kann mit unterschiedlichen Verfahren gemessen werden. Das einfachste ist z.B. das Lysimeter. Dabei wird die Gewichtsänderung der Pflanze zusammen mit dem Pflanztopf auf einer Waage stehend kontinuierlich bestimmt. Der Gewichtsverlust wird als Transpiration angesehen.

Ein weiteres wichtiges Verfahren ist die Bestimmung des Xylemflusses (US 4,745,805, DE 102 22 640, US 5,269,183 A).

Sowohl Lysimeter als auch Xylemfluss geben die Transpiration der Gesamtpflanze an. Kenntnis über die unmittelbare pflanzliche Regulation des Wasserverbrauchs am eigentlichen Ort des Geschehens, d.h. auf Blattebene, ist jedoch für die oben genannten Anwendungen von großem Wert. Genauere Information zur Transpiration auf Blattebene lässt sich jedoch methodisch bedingt weder aus Lysimeter- noch aus Xylemflussdaten ableiten.

Die meist gebräuchliche Methode zur Bestimmung der Wasserabgabe auf Blattebene ist die Gaswechselmessung mittels Gasküvette. Dabei wird das jeweilige Transpirationsorgan bzw. Objekt (Blatt, Nadel oder Rinde) in ein durchsichtiges Gefäß eingeschlossen. Man bestimmt die Zunahme der Luftfeuchtigkeit in der Luft bzw. in der Abluft des Gefäßes, woraus sich die Transpiration pro Blattfläche errechnen lässt (DE3032833C2). Der Nachteil des Verfahrens ist die massive Veränderung der natürlichen physikalischen Umgebung der betrachteten Transpirationsorgane. Aufgrund dieser Veränderung der physikalischen Umgebungsbedingungen ist es nicht zu vermeiden, dass sich dabei eine große Abweichung von der tatsächlichen Transpiration ergibt.

Ein bekanntes, aber in der Vergangenheit wenig beachtetes Verfahren ist die Messung des Temperaturunterschieds zwischen dem Blatt und einem nicht transpirierenden Referenzkörper (Delta-T(ref-leaf)). Bei der Transpiration geht Wasser im Blatt in gasförmige Form über und kühlt das Blatt dadurch ab, während beim Referenzköper die Abkühlung nicht stattfindet. Der Temperaturunterschied zwischen den beiden spiegelt damit die Transpirationsintensität wider (Impens I. I. et al.: Diffusive resistances at, and transpiration rates from leaves in situ within the vegetative canopy of a corncrop. Plant physiology, 1967, 42 Jg., Nr.1, S. 99-104).

Zum Messen des Temperaturunterschieds werden z.B. Thermoketten eingesetzt (Haines, F.M.: Transpiration and Pressure Deficit, III. Oberservations by the Thermopile Method. Annals of Botany, 1936, Nr. 1, S. 1-22). In den letzten Jahren erfolgt die Temperaturmessung über Thermographie (Garbe, C., Schurr, U., & Jaehne, B.: Thermographic measurements on plant leaves. 2002, Proc. SPIE, Vol. 4710, pp. 407-416).

Das Delta-T(ref-leaf)-Verfahren verursacht nur eine geringe Störung in der natürlichen Umgebungsbedingungen und erlaubt somit die kontinuierliche Messung der Transpiration unter realistischen Bedingungen. Ein weiterer wichtiger Vorteil des Verfahrens ist dessen vergleichsweise einfach und kostengünstige Umsetzung. Trotz der genannten Vorteile findet es aber keine verbreitete Anwendung in der Praxis. Ein Grund dafür ist die komplexe Interpretation des Messsignals aufgrund der Interaktion des Messsignals mit Umweltfaktoren, vor allem Wind an der Blattoberfläche. Es ist offensichtlich, dass Delta-T(ref-leaf) durch Wind beeinflusst wird. Bei gleicher Transpirationsrate führt stärkerer Wind zu einem geringeren Temperaturunterschied Delta-T(ref-leaf). Die praktische Anwendung des Delta-T(ref-leaf)-Verfahrens ist daher nur möglich, wenn zusätzlich die Windgeschwindigkeit an der Blattoberfläche parallel gemessen wird. Bislang ist dies aber mit vertretbarem Aufwand nicht möglich.

Die Aufgabe der vorliegenden Erfindung liegt darin, mit konstruktiv möglichst einfachen Mitteln eine Vorrichtung und ein Verfahren zum zuverlässigen Ermitteln einer Transpirationsrate eines Objekts zur Verfügung zu stellen.

Diese Aufgabe wird gelöst durch eine Vorrichtung mit den Merkmalen des Anspruchs 1 oder durch ein Verfahren mit den Merkmalen des Anspruchs 5. Vorteilhafte Weiterbildungen der Erfindung sind in den abhängigen Ansprüchen angegeben.

Die Erfindung beruht auf der überraschenden Idee, neben einer ersten Temperaturdifferenz zum Vergleichen des Objekts (bzw. der Probe) und des idealerweise transpirationsfreien Referenzkörpers eine zweite Temperaturdifferenz zwischen den Temperaturen an der Oberfläche des Objekts bzw. des Referenzkörpers und einem davon etwas beabstandeten Messpunkts zu ermitteln. Diese zweite Temperaturdifferenz wird nun als zur Windgeschwindigkeit an der Objektoberfläche proportionales Maß herangezogen, um anhand der beiden gemessenen Temperaturdifferenzen die Transpirationsrate des Objekts bestimmen zu können. Als Datenrecorder zum Aufzeichnen oder Berechnen der ersten und zweiten Temperaturdifferenzen kann ein Computer verwendet werden. Alternativ könnte der Datenrecorder allein eine Aufzeichnungsfunktion bieten und z. B. an einen Computer zum Auswerten der Daten angeschlossen sein.

Vorzugsweise werden bei der Vorrichtung Temperaturfühler, insbesondere Thermistoren, zum Messen der Temperaturen vorgesehen.

Alternativ könnte zum Messen der ersten und/oder der zweiten Temperaturdifferenz jedoch auch jeweils ein Thermoelement wie eine Thermokette verwendet werden, wie sie beispielsweise in der DE 10 2015 120 899 A1 beschrieben ist. Damit eröffnet die Erfindung die Möglichkeit, statt absoluter Temperaturen direkt Temperaturdifferenzen zu messen.

In einer vorteilhaften Weiterbildung ist ein Ausgleichskörper thermisch mit der Thermokette verbunden. Dieser Ausgleichskörper sollte eine mittlere bis hohe Wärmeleitfähigkeit aufweisen. Er ermöglicht es, statt einer lokalen Temperatur eine Durchschnittstemperatur eines größeren Bereichs der Oberfläche eines Objekts zu messen, was insbesondere bei Objekten mit inhomogenen thermischen Eigenschaften vorteilhaft sein kann.

Die Erfindung bezieht sich auch auf ein Verfahren zum Ermitteln einer Transpirationsrate eines Objekts. Dieses umfasst die folgenden Schritte:

- Bestimmen einer ersten Temperaturdifferenz zwischen den Temperaturen an einer Oberfläche des Objekts und an einer Oberfläche eines Referenzkörpers,

- Bestimmen einer zweiten Temperaturdifferenz zwischen den Temperaturen an der Oberfläche des Objekts und an einem von der Oberfläche des Objekts beabstandeten ersten Messpunkt, oder zwischen den Temperaturen an der Oberfläche des Referenzkörpers und an einem von der Oberfläche des Referenzkörpers beabstandeten zweiten Messpunkt,

- Ermitteln der Transpirationsrate des Objekts unter Berücksichtigung der ersten und der zweiten Temperaturdifferenz.

Die zweite Temperaturdifferenz wird damit gleichsam als Indikator für die Windgeschwindigkeit an der Grenze zwischen der Oberfläche des Objekts bzw. des Referenzkörpers und der Umgebungsluft herangezogen, um dann im Zusammenspiel mit der ersten Temperaturdifferenz die Transpirationsrate des Blattes unabhängig von der jeweils herrschenden Windgeschwindigkeit ermitteln zu können.

Vorteilhaft ist es, wenn das Ermitteln der Transpirationsrate des Objekts unter Berücksichtigung einer Kalibrierung erfolgt. Eine solche Kalibrierung kann beispielsweise anhand einer gravimetrischen Messung der Transpiration oder der Transpirationsrate des Objekts oder eines Vergleichsobjekts erfolgen. Denkbar wäre es dafür, den Messaufbau auf eine möglichst präzise Waage zu stellen, um gravimetrisch (d. h. durch Veränderung des Gewichts) den Wasserverlust und damit die Transpiration und gleichzeitig die zwei Temperaturdifferenzen zu messen. Aus der Veränderung oder dem zeitlichen Verlauf dieser Messdaten können dann beispielsweise deterministisch oder statistisch die Parameter der Transpiration kalibriert werden.

Bei der Kalibrierung kann z.B. kontinuierlich der gravimetrisch ermittelte Transpirationsverlust des Blattes/des Nadelzweiges (in kg Wasser pro Zeit, umgerechnet in Stoffmenge Mol, über molare Masse H2O) den kontinuierlich aufgezeichneten Sensordaten gegenübergestellt werden.

Als vorteilhaft hat es sich erwiesen, wenn der erste Messpunkt maximal 10 cm von der Oberfläche des Objekts entfernt oder der zweite Messpunkt maximal 10 cm von der Oberfläche des Referenzkörpers entfernt ist. Auf diese Weise wird sichergestellt, dass tatsächlich ein Maß für die Windgeschwindigkeit am Ort des Objekts bzw. des Referenzkörpers Berücksichtigung findet.

Besonders geeignet ist das Verfahren, wenn als Objekt ein Pflanzenblatt oder eine Nadel einer Pflanze verwendet wird. Das Verfahren lässt sich allerdings auch auf andere transpirierende Objekte anwenden.

Für besonders präzise Messungen ist es vorteilhaft, wenn sich das Objekt und der Referenzkörper im Hinblick auf ihre Geometrie, ihre Licht- und/oder ihre Wärmeeigenschaften möglichst ähnlich sind. Konkret lässt sich dies erreichen, indem sich das Objekt und der Referenzkörper im Hinblick auf eine Abmessung, im Hinblick auf ein Volumen, im Hinblick auf einen Absorptionskoeffizienten bei einer bestimmten Wellenlänge oder in einem bestimmten Wellenlängenbereich, im Hinblick auf ihre Wärmekapazität und/oder im Hinblick auf ihren Wärmeleitungskoeffizienten um jeweils maximal 20 % unterscheiden, vorzugsweise um maximal 10 %.

Im Folgenden werden vorteilhafte Ausführungsformen der Erfindung anhand einer Zeichnung näher erläutert. Im Einzelnen zeigen

Fig. 1    eine schematische Darstellung einer ersten Ausführungsform;

Fig. 2    eine schematische Darstellung einer zweiten Ausführungsform;

Fig. 3    eine schematische Darstellung einer dritten Ausführungsform; und

Fig. 4    eine schematische Darstellung einer vierten Ausführungsform.

Einander entsprechende Komponenten sind in den Figuren durchgängig mit gleichen Bezugszeichen versehen.

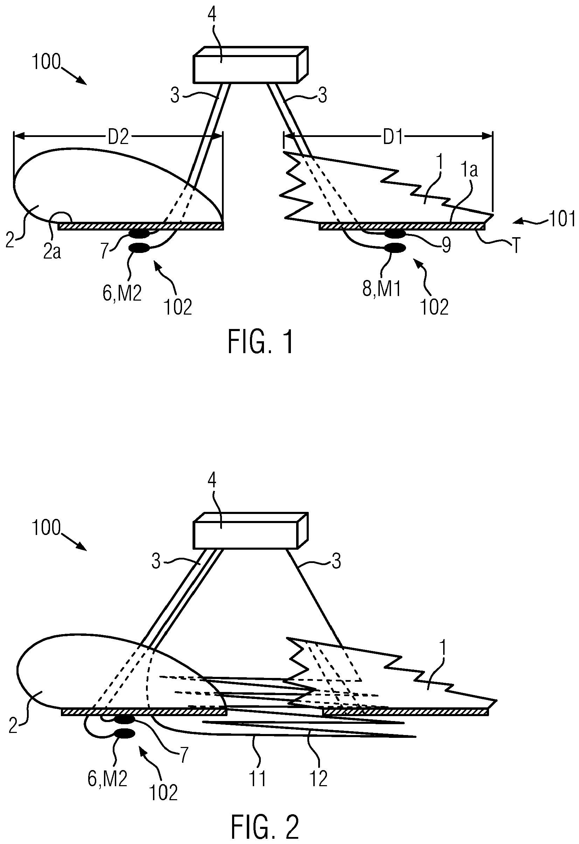

Fig. 1 zeigt eine erste Vorrichtung 100 zum Ermitteln einer Transpirationsrate eines Objekts 1, bei dem es sich um ein Pflanzenblatt 1 handelt, das beispielsweise auf einem Träger T liegen oder befestigt sein kann.

Ein in der Geometrie und in den Licht- und Wärmeeigenschaften blattähnlicher, aber nicht transpirierender Referenzkörper 2 ist in einem Abstand von wenigen Millimetern bis maximal ca. 50 cm zu dem Blatt 1 platziert. Mit "in der Geometrie und in den Licht- und Wärmeigenschaften blattähnlich" ist im Kontext der Erfindung gemeint, dass sich das Objekt 1 und der Referenzkörper 2 im Hinblick auf eine Abmessung, im Hinblick auf einen Absorptionskoeffizienten bei einer bestimmten Wellenlänge oder in einem bestimmten Wellenlängenbereich, im Hinblick auf eine Wärmekapazität ihres Materials und/oder im Hinblick auf einen Wärmeleitungskoeffizienten ihres Materials oder ihrer Oberfläche um jeweils maximal 20 % voneinander unterscheiden, vorzugsweise um maximal 10 %. Beispielsweise kann eine Abmessung D2 des Referenzkörpers 2 um bis zu 20 % von einer entsprechenden Abmessung D1 des Objekts 1 abweichen. Die Abweichung kann beispielsweise jeweils ausgehend von demjenigen Gegenstand 1, 2 betrachtet werden, bei dem die jeweilige Größe den größeren Wert hat. Dahinter steht das Ziel, dass der Referenzkörper 2 und das Objekt 1 sich im Hinblick auf eine oder mehrere Eigenschaften möglichst ähnlich sind. Dies gilt jedoch nicht im Hinblick auf die Transpiration. Der Referenzkörper 2 ist idealerweise überhaupt nicht transpirierend, weist also beispielsweise keinen Wasseranteil auf. Im Ausführungsbeispiel der Fig. 1 befindet sich der Referenzkörper 2 auf einem eigenen Träger, der jedoch optional ist oder bei dem es sich auch um denselben Träger T für das Objekt 1 handeln kann.

Die Temperaturen am Blatt 1 (Tl), an der Referenz (Tr), sowie die Temperatur in Blattnähe (Tla) bzw. in Referenznähe (Tra) werden nun vorzugsweise parallel gemessen. Der Delta-T(ref-leaf) ergibt sich aus der Differenz zwischen den Temperaturen Tl und Tr. Der Temperaturunterschied zwischen Tl und Tla sowie Tr und Tra zeigt die Intensität, wie stark die Luft an der Grenzschicht durch Wind abgetragen wird. Er ist somit ein Indikator über die Windgeschwindigkeit an der Grenze Oberfläche-Umgebungsluft des Blattes 1 bzw. der Referenz 2. Die Transpirationsrate des Blattes 1 lässt sich mit folgender Gleichung errechnen:

$$W=f(Delta\text{-}T(ref\text{-}leaf), (Tr\text{-}Tra))\ bzw.$$

$$W=f(Delta\text{-}T(ref\text{-}leaf), (Tl\text{-}Tla))$$

Die Parameter dieser Gleichungen lassen sich vor der eigentlichen Messung z. B. in Form einer Kalibrierung gravimetrisch ermitteln. Stellt man den Aufbau auf eine sehr präzise Waage und misst somit gravimetrisch (d. h. über den Gewichtsverlust über eine bestimmte Zeit) die Transpiration und die vier Temperaturen parallel, so lassen sich die Parameter z. B. deterministisch bzw. statistisch bestimmen.

Ein konkretes Beispiel für eine solche Gleichung könnte wie folgt lauten:

$$W = a+b*(Tr\text{-}Tl)+c*(Tl\text{-}Tla)+d*(Tr\text{-}Tra)$$

Parameter für Kalibrierfunktion, ermittelt via gravimetrischer Vergleichsmessung an Fichte, unter variablen Umgebungsbedingungen (Strahlung, Lufttemperatur, Luftfeuchtigkeit):
Bestimmtheitsmaß der Kalibrierfunktion Rsq: 94,4%

a = +0.000274455

b = +0.452055039

c = -0.005186585

d = -0.000632594

Die Vorrichtung 100 in Fig. 1 umfasst eine erste Anordnung 101 zum Bestimmen einer ersten Temperaturdifferenz zwischen den Temperaturen an der Oberfläche 1a des Objekts 1 und an der Oberfläche 2a des Referenzkörpers 2. Diese erste Anordnung 101 umfasst einen ersten Temperaturfühler 9, der den Träger T oder noch besser direkt die Oberfläche 1a des Objekts 1 kontaktiert und dessen Temperatur Tl misst, sowie einen zweiten Temperaturfühler 7, der direkt die Temperatur an der Oberfläche 2a des Referenzkörpers 2 misst. Ferner umfasst die Vorrichtung 100 eine zweite Vorrichtung 102 zum Bestimmen einer zweiten Temperaturdifferenz. Hierzu sind zwei verschiedene Varianten in Fig. 1 dargestellt. In der einen Variante umfasst die zweite Anordnung 102 den ersten Temperaturfühler 9 zum Messen der Temperatur an der Oberfläche 1a des Objekts 1 sowie einen weiteren Temperaturfühler 8 an einem ersten Messpunkt M1. Der erste Messpunkt M1 ist dabei vom ersten Temperaturfühler 9 beabstandet, allerdings vorzugsweise nicht weiter als 10 cm. Als zweite Temperaturdifferenz wird bei dieser Anordnung 102 die Differenz der von den beiden Temperaturfühlern 8, 9 gemessenen Temperaturen ermittelt.

In einer zweiten Variante umfasst die Anordnung 102 den Temperaturfühler 7 zum Messen der Temperatur an der Oberfläche 2a des Referenzkörpers 2 sowie einen weiteren Temperaturfühler 6, der die Temperatur an einem zweiten Messpunkt M2 misst. Dieser zweite Messpunkt M2 ist vom Temperaturfühler 7 und der Oberfläche 2a des Referenzkörpers 2 beabstandet, allerdings vorzugsweise nicht weiter als 10 cm.

Der Temperaturunterschied zwischen Blatt 1 und Referenz 2 Delta-T(ref-leaf) wird in Fig. 1 mit Temperaturfühlern 7 (Tr) und 9 (Tl) gemessen, die blattnahe Temperatur (Tla) und die referenznahe Temperatur (Tra) mit zwei weiteren Temperaturfühlern 6, 8 gemessen. Die Daten werden über Kabel 3 an den Datenrekorder 4 übertragen. Der Temperaturrekorder 4 kann ein Computer sein.

Fig. 2 zeigt eine weitere mögliche Ausführung.

Hier wird statt Temperaturfühlern für eine absolute Messung eine Thermokette 11 mit Windungen 12 für eine direkte Temperatur-Differenzmessung eingesetzt. Die Thermokette 11 erfasst mehrere Messpunkte und trägt somit der thermischen Inhomogenität des Blattes 1 Rechnung. Der Temperaturunterschied zwischen dem Referenzkörper 2 und der referenznahen Luft (Tr-Tra) wird mit zwei Thermistoren als Temperaturfühlern 6 und 7 gemessen.

Es ist offensichtlich, dass man die Temperaturfühler 7 und 6 auch auf der Seite des Blattes 1 platzieren kann. Aufgrund der unterschiedlichen thermischen Eigenschaften wird die Kalibrationskurve dann anders ausfallen.

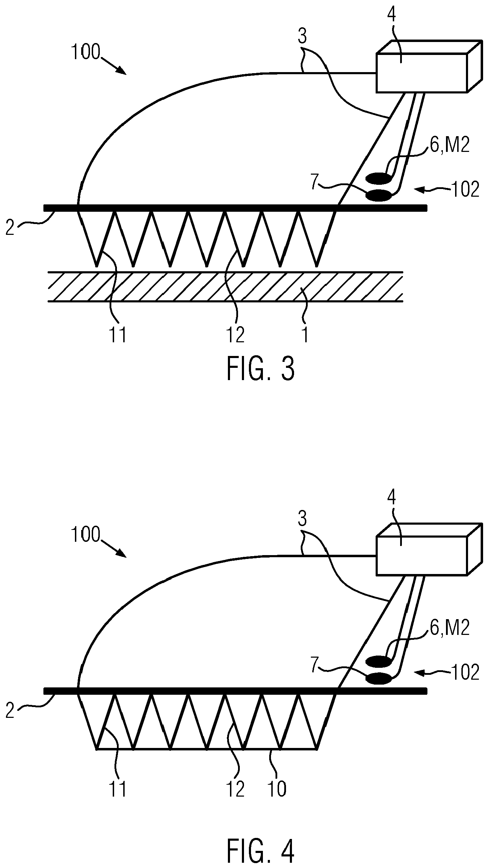

Analog zum Sensor für ein Laubblatt kann man einen ähnlichen Sensor für Pflanzenarten mit Nadelblättern 1 als Objekt herstellen. Fig. 3 zeigt eine der möglichen Varianten für Nadelblätter. Dabei werden die Messpunkte der Thermokette 11 mit Windungen 12 am Referenzkörper 2 angeordnet, der ähnliche thermische Eigenschaften aufweist wie die Nadeln 1. Die andere Seite der Thermokette 11 wird in das Nadelbüschel 1 eingefügt, sie erfasst an mehreren Stellen die Temperatur im Innenraum der Nadelbüschel 1. Die absolute Temperatur an der Referenz (Tr) und in der referenznahen Luft (Tra) wird über Temperaturfühler 6 und 7 erfasst.

Für Blätter 1 und Nadeln 1, die eine inhomogene thermische Eigenschaft aufweisen, können die Thermoketten 11 in Fig. 2 und 3 die richtige Blatttemperatur nur schwer exakt erfassen. In dem Fall wird, wie in Fig. 4 gezeigt, ein Ausgleichskörper 10 an einer Blattseite angebracht. Der Ausgleichskörper 10 weist eine möglichst gute Wärmeleitfähigkeit auf und spiegelt die Durchschnittstemperatur des Blattes 1 bzw. der Nadel 1 wider, überträgt dies an die Thermokette 11.

**Patentansprüche**

1. Vorrichtung (100) zum Ermitteln einer Transpirationsrate eines Objekts (1), insbesondere eines Pflanzenblatt (1) oder einer Pflanzen-Nadel (1), wobei die Vorrichtung (100) Folgendes umfasst:

   einen Referenzkörper (2),
   eine Anordnung (101) zum Bestimmen einer ersten Temperaturdifferenz zwischen den Temperaturen an einer Oberfläche (1a) des Objekts (1) und an einer Oberfläche (2a) des Referenzkörpers (2),
   eine Anordnung (102) zum Bestimmen einer zweiten Temperaturdifferenz zwischen den Temperaturen an der Oberfläche (1a) des Objekts (1) und an einem von der Oberfläche (1a) des Objekts (1) beabstandeten ersten Messpunkt (M1), oder zwischen den Temperaturen an der Oberfläche (2a) des Referenzkörpers (2) und an einem von der Oberfläche (2a) des Referenzkörpers (2) beabstandeten zweiten Messpunkt (M2), sowie
einen Datenrekorder (4) zum Aufzeichnen oder Berechnen der ersten und zweiten Temperaturdifferenzen.

2. Vorrichtung nach Anspruch 1, **dadurch gekennzeichnet, dass** die Vorrichtung (100) Temperaturfühler (6 - 9), insbesondere Thermistoren (6 - 9), zum Messen der Temperaturen aufweist.

3. Vorrichtung nach einem der vorangehenden Ansprüche, **dadurch gekennzeichnet, dass** die Vorrichtung zum Messen der ersten oder der zweiten Temperaturdifferenz eine Thermokette (11) aufweist.

4. Vorrichtung nach Anspruch 3, **dadurch gekennzeichnet, dass** ein Ausgleichskörper (10) thermisch mit der Thermokette (11) verbunden ist.

5. Verfahren zum Ermitteln einer Transpirationsrate eines Objekts (1), umfassend die folgenden Schritte:

   Bestimmen einer ersten Temperaturdifferenz zwischen den Temperaturen an einer Oberfläche (1a) des Objekts (1) und an einer Oberfläche (2a) eines Referenzkörpers (2), Bestimmen einer zweiten Temperaturdifferenz zwischen den Temperaturen an der Oberfläche (1a) des Objekts (1) und an einem von der Oberfläche (1a) des Objekts (1) beabstandeten ersten Messpunkt (M1), oder zwischen den Temperaturen an der Oberfläche (2a) des Referenzkörpers (2) und an einem von der Oberfläche (2a) des Referenzkörpers (2) beabstandeten zweiten Messpunkt (M2), Ermitteln der Transpirationsrate des Objekts (1) unter Berücksichtigung der ersten und der zweiten Temperaturdifferenz.

6. Verfahren nach Anspruch 5, **dadurch gekennzeichnet, dass** das Ermitteln der Transpirationsrate des Objekts (1) unter Berücksichtigung einer Kalibrierung erfolgt.

7. Verfahren nach einem der Ansprüche 5 oder 6, **dadurch gekennzeichnet, dass** die Kalibrierung anhand einer gravimetrischen Messung der Transpiration oder der Transpirationsrate erfolgt.

8. Verfahren nach einem der Ansprüche 5 bis 7, **dadurch gekennzeichnet, dass** der erste Messpunkt (M1) maximal 10 cm von der Oberfläche (1a) des Objekts (1) entfernt oder der zweite Messpunkt (M2) maximal 10 cm von der Oberfläche (2a) des Referenzkörpers (2) entfernt ist.

9.  Verfahren nach einem der Ansprüche 5 bis 8, **dadurch gekennzeichnet, dass** das Objekt (1) ein Pflanzenblatt oder eine Pflanzen-Nadel ist.

10. Verfahren nach einem der Ansprüche 5 bis 9, **dadurch gekennzeichnet, dass** das Objekt (1) und der Referenzkörper (2) sich

    - im Hinblick auf eine Abmessung (D1, D2),
    - im Hinblick auf einen Absorptionskoeffizienten bei einer bestimmten Wellenlänge oder in einem bestimmten Wellenlängenbereich,
    - im Hinblick auf eine Wärmekapazität und/oder
    - im Hinblick auf einen Wärmeleitungskoeffizienten

    um jeweils maximal 20%, vorzugsweise um maximal 10%, voneinander unterscheiden.

FIG. 1

FIG. 2

FIG. 3

FIG. 4

**Europäisches Patentamt**
**European Patent Office**
**Office européen des brevets**

# EUROPÄISCHER RECHERCHENBERICHT

Nummer der Anmeldung

EP 20 18 4942

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | HAINES F M: "Transpiration and Pressure Deficit. III. Observations by the Thermopile Method", ANNALS OF BOTANY, ACADEMIC PRESS, LONDON, GB, Bd. OS-50, Nr. 1, 1. Januar 1936 (1936-01-01), Seiten 1-22, XP008183973, ISSN: 0305-7364, DOI: 10.1093/OXFORDJOURNALS.AOB.A090575 * Experimental method; Seite 7 - Seite 8 * * Control experiments; Seite 8 - Seite 9 * ----- | 1-10 | INV. G01N33/00 |
| X | CHRISTOPH GARBE ET AL: "Thermographic measurements on plant leaves", PROCEEDINGS OF SPIE, Bd. 4710, 15. März 2002 (2002-03-15), Seiten 407-416, XP055764475, 1000 20th St. Bellingham WA 98225-6705 USA ISSN: 0277-786X, DOI: 10.1117/12.459590 ISBN: 978-1-5106-3927-0 * 3.2 Passive thermography * ----- | 1-10 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 13. Januar 2021 | Marzocchi, Olaf |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4745805 A
- DE 10222640
- US 5269183 A
- DE 3032833 C2
- DE 102015120899 A1


**In der Beschreibung aufgeführte Nicht-Patentliteratur**

- **IMPENS I. I. et al.** Diffusive resistances at, and transpiration rates from leaves in situ within the vegetative canopy of a corncrop. *Plant physiology,* 1967, vol. 42 (1), 99-104
- **HAINES, F.M.** Transpiration and Pressure Deficit, III. Oberservations by the Thermopile Method. *Annals of Botany,* 1936, 1-22
- **GARBE, C. ; SCHURR, U. ; JAEHNE, B.** Thermographic measurements on plant leaves. *Proc. SPIE,* 2002, vol. 4710, 407-416